(19) Europäisches Patentamt
European Patent Office
Office européen des brevets

(11) Publication number: **0 050 286**
**B1**

(12) # EUROPEAN PATENT SPECIFICATION

(45) Date of publication of patent specification: **22.05.85**

(21) Application number: **81108212.2**

(22) Date of filing: **12.10.81**

(00) **Consolidated with 81903256.6 (European application No.) by decision dated 18.11.83.**

(51) Int. Cl.⁴: **C 07 C 129/12,**
C 07 C 149/437,
C 07 D 213/75,
C 07 D 295/12,
C 07 D 213/40,
C 07 D 211/56,
C 07 D 265/30,
C 07 D 309/14, A 61 K 31/325

(54) **Guanidine derivatives and pharmaceutical compositions containing them.**

(30) Priority: **20.10.80 US 198424**

(43) Date of publication of application:
**28.04.82 Bulletin 82/17**

(45) Publication of the grant of the patent:
**22.05.85 Bulletin 85/21**

(84) Designated Contracting States:
**AT BE CH DE FR GB IT LI LU NL SE**

(56) References cited:
**US-A-3 896 160**

(73) Proprietor: **SCHERING CORPORATION**
**2000 Galloping Hill Road**
**Kenilworth, New Jersey 07033 (US)**

(72) Inventor: **Nafissi-Varchei, Mohammad Mehdi**
**15 Grandview Ave.**
**North Caldwell New Jersey 07006 (US)**

(74) Representative: **von Kreisler, Alek et al**
**Patentanwälte Von Kreisler-Schönwald-Fues-Keller Selting-Werner Deichmannhaus am Hauptbahnhof**
**D-5000 Köln 1 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 050 286 B1**

Courier Press, Leamington Spa, England.

**Description**

This invention relates to novel guanidine derivatives of the general formula

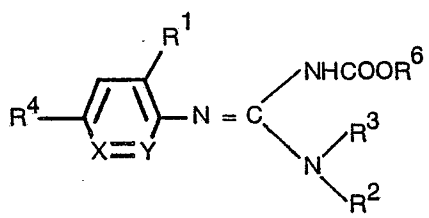

(I) ,

their isomers at the guanidine groups and pharmaceutically acceptable salts of such compounds, wherein

$R^6$ is $C_{1\ to\ 6}$ alkyl or $C_{1\ to\ 6}$ alkyl substituted by $C_{1\ to\ 6}$ alkoxy, hydroxy or amino;

one of X and Y is CR and the other is CH or N;

$R^1$ is hydrogen, halogen, nitro, amino, $NHCOR^8$ or $NHSO_2R^8$;

$R^2$ and $R^3$ are independently selected from hydrogen, N-alkyl-piperidyl (wherein the alkyl group has 1 to 4 carbon atoms), tetrahydropyranyl, morpholinyl, piperidyl and $C_1$ to $C_6$ alkyl substituted by one or more substituents selected from OH, $SCH_3$, $OSO_3H$, $SO_3H$, $COR^9$, $COOR^{10}$, piperazinyl, pyridyl and

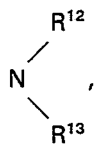

,

with the proviso that $R^2$ and $R^3$ cannot both be hydrogen;

one of $R^4$ and $R^5$ is selected from hydrogen, $C_1$ to $C_6$ alkyl and $C_1$ to $C_6$ alkoxy and the other from hydrogen, $C_1$ to $C_6$ alkyl and $R^{14}Z$ wherein $R^{14}$ is $C_1$ to $C_6$ alkyl or phenyl and Z represents O, S, $SO_n$ or $SO_2NR^{15}$ with n being 1, 2 or 3 and $R^{15}$ being hydrogen or $C_1$ to $C_6$ alkyl; whereby, in the above definitions,

$R^8$ is $C_1$ to $C_6$ alkyl, $C_1$ to $C_6$ alkyl substituted with phenyl or halogen, phenyl or phenyl substituted with $C_1$ to $C_6$ alkyl or with halogen;

$R^9$ is $C_1$ to $C_6$ alkyl, $C_1$ to $C_6$ acyl or a peptide residue having up to three amino acids;

$R^{10}$ is hydrogen or $C_1$ to $C_6$ alkyl;

$R^{12}$ and $R^{13}$ are independently selected from hydrogen, $C_1$ to $C_6$ alkyl,

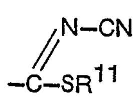

and

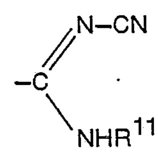

with $R^{11}$ being $C_1$ to $C_6$ alkyl.

The $C_1$ to $C_6$ alkyl and alkoxy groups referred to above may be straight or branched chains and include, when the groups have 3 or more C-atoms, cycloalkyl and, when the groups have 4 to 6 C-atoms, also cycloalkylalkyl groups. The tetrahydropyranyl, morpholinyl, piperidyl, piperazinyl, and pyridyl groups include all possible configurations (e.g. 2- 3- and 4-pyridyl). Halogen includes fluorine, chlorine, bromine and iodine. The $SO_n$-group in the definition of $R^4$ and/or $R^5$ includes all possible variations, e.g. $—OSO_2—$ and $—SO_2O—$. Equally the $SO_2NR^{15}—$ group may be $—SO_2NR^{15}—$ or $—NR^{15}SO_2—$.

The preferred definition of $R^2$ is hydrogen and it is equally preferred that only one of $R^4$ and $R^5$ represents hydrogen and the other is as defined above (except hydrogen). A more important group of compounds is the one wherein $R^4$ is hydrogen, X is $CR^5$ (with $R^5$ being other than hydrogen) and Y is N or CH, preferably CH. The preferred meaning of $R^1$ is $NO_2$, $NHCOR^8$ or $NH_2$, and of $R^3$ is a substituted $C_1$ to $C_6$ alkyl group wherein the preferred substituents are $COOR^{10}$, piperazinyl, pyridyl, $NR^{12}R^{13}$, $SO_3H$ and $OSO_3H$ ($R^{10}$, $R^{12}$ and $R^{13}$ being as defined above). The preferred definition of $R^4$ and $R^5$ is $R^{14}Z$ with $R^{14}$ being $C_1$ to $C_6$ alkyl or phenyl, particularly $C_1$ to $C_6$ alkyl and Z being as defined above, but preferably S.

The preferred meaning of $R^6$ is $CH_3$.

The most preferred group of compounds of this invention may be illustrated by the following formula

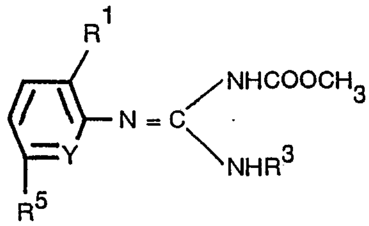

(II)

wherein

$R^1$ is $NO_2$, $NH_2$ or $NHCOR^8$;

$R^3$ is $C_1$ to $C_3$ alkyl substituted by

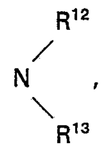 ,

piperazinyl, $COOR^{10}$, $SO_3H$ or $OSO_3H$;

$R^5$ is $—S{\cdot}CH_2(CH_2)_mCH_3$ (m being 0, 1, 2 or 3, preferably 1);

$R^8$ is $C_1$ to $C_3$ alkyl, preferably $CH_3$;

$R^{10}$ is hydrogen or $C_1$ to $C_3$ alkyl, preferably hydrogen; and

$R^{12}$ and $R^{13}$ are either both $C_1$ to $C_3$ alkyl, preferably ethyl, or one is hydrogen and the other is

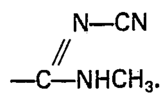.

The compounds of this invention may be prepared according to processes generally known in the art for the preparations of similar compounds. Preferably the compounds are obtained according to the reaction illustrated in the following reaction scheme:

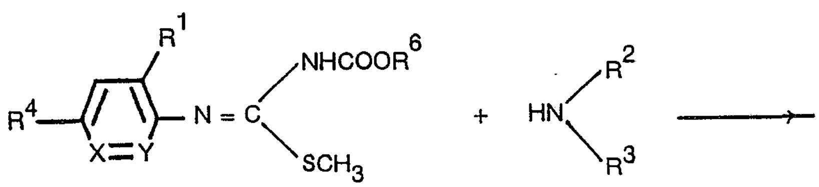

I

(III) (IV)

($R^1$ to $R^6$, X and Y are as defined above).

The reaction is preferably carried out by dissolving the reactants III and IV in an inert solvent, if necessary by heating, and to stir the resulting solution, preferably at ambient temperature, until the reaction is completed.

The starting compounds of formula III are either known or may be obtained by standard reactions well known in the art. The compounds of formula III, for example, may be obtained by following the procedure outlined in Belgian Patent No. 654.306 for the preparation of closely related compounds, i.e.:

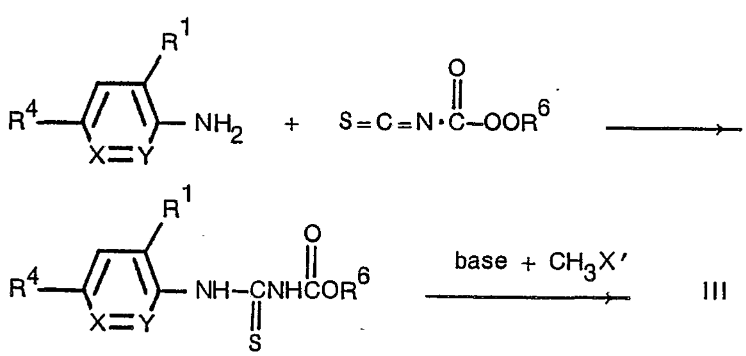


III

($R^1$, $R^4$, $R^6$ X and Y are as defined above and X′ represents halogen (Cl, Br, or I) or sulfonate).

Included within the scope of this invention are the isomers at the guanidine groups e.g.

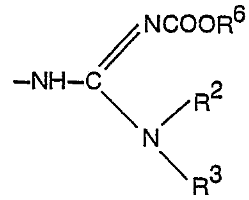

and the salts.

This includes acid addition salts and (metal) salts of those compounds wherein $R^2$ and/or $R^3$ represent $C_1$ to $C_6$ alkyl substituted by $COOR^{10}$ with $R^{10}$ being hydrogen, or by $OSO_3H$ or $SO_3H$. Preferred metal salts are alkali metal salts, in particular sodium salts. The salts are obtained according to standard procedures. Typical acids to be used for forming acid addition salts are hydrochloric, hydrobromic, sulfuric, phosphoric, acetic, propionic, succinic, pamoic, caproic, palmitic and stearic acid.

The following example illustrates the process of this invention.

Example

N-Methoxycarbonyl-N′-[2-(N′-cyano-N″-methyl guanidino)ethyl]-N″-(2-nitro-4-propylthiophenyl)guanidine.

Dissolve 6.7 g of N-methoxycarbonyl-N′-(2-nitro-4-propylthiophenyl)-S-methyl-isothiourea and 3.7 g of N′-(2-aminoethyl)-N′-cyano-N″-methyl-guanidine in 50 ml of acetonitrile by heating. Stir the resulting solution for 24 hours at ambient temperature. Remove the solvent by evaporation and chromatograph the residue on 350 g of silica gel, eluting with 1% methanol in methylene chloride to give, after evaporation of the solvent, the title product (m.p. 72—73°C).

By following the process exemplified above, using the appropriate starting compounds, the compounds listed and tabulated below may be prepared:

N-methoxycarbonyl-N′-(2-nitro-5-propylthiophenyl)-N″-[2-(N-piperazinyl)ethyl]guanidine (m.p. 101—104°C);

N-methoxycarbonyl-N′-[2(N′-cyano-N″-methylguanidino)ethyl]-N-″-[2-nitro-5-propylthiophenyl]guanidine (m.p. 124°C, dec.);

N-methoxycarbonyl-N′-(2-nitro-5-propylthiophenyl)-N″-(2-diethylaminoethyl)guanidine(amber gum);

N-methoxycarbonyl-N′-(2-nitro-5-propylthiophenyl)-N″-(2-carboxyethyl)guanidine (m.p. 116—118°C, dec.);

N-methoxycarbonyl-N′-[2-(N′-cyano-N″-methylguanidino) ethyl]-N″-(2-amino-5-propylthiophenyl)guanidine(m.p. 116—119°C, dec.);

N-methoxycarbonyl-N′-[2-(N′-cyano-N″-methylguanidino)ethyl]-N″-[2-acetamido-5-propylthiophenyl]guanidine (m.p.>160°C, dec.);

N-methoxycarbonyl-N′-(3-carboxypropyl)-N″-(2-nitro-5-propylthiophenyl)guanidine (m.p. 112—114°C, dec.);

N-methoxycarbonyl-N′-carboxymethyl-N″-(2-nitro-5-propylthiophenyl)guanidine sodium salt (m.p. 195—198°C, dec.);

N-methoxycarbonyl-N′-(2-diethylaminoethyl)-N″-(2-nitro-5-propoxyphenyl)guanidine (m.p. 60—63°C);

N-methoxycarbonyl-N′-[2-(N′-cyano-N″-methylguanidino)ethyl]-N″-(2-nitro-5-propoxyphenyl)guanidine (m.p. 149—152°C, dec.);

N-methoxycarbonyl-N′-(2-diethylaminoethyl)-N″-(2-nitro-4-propylthiophenyl)guanidine (orange viscous oil);

N-methoxycarbonyl-N′-(2-diethylaminoethyl)-N″-(2-nitro-5-propylthiophenyl)guanidine;

N-methoxycarbonyl-N′-[2-(N′-cyano-N″-methylguanidino)ethyl]-N″-(2-nitro-5-phenylthiophenyl)guanidine (m.p. 181—183°C, dec.);

N-methoxycarbonyl-N′-(2-nitro-4-propyloxyphenyl-N″-(2-diethylaminoethyl)guanidine (m.p. 91—93°C);

N-methoxycarbonyl-N′-[2-(N′-cyano-N″-methylguanidino)ethyl]-N″-(2-acetamido-4-methyl-5-propoxyphenyl)guanidine (m.p. 225°C, dec.);

N-methoxycarbonyl-N′-(2-pyridylmethyl)-N″-(2-nitro-4-propoxyphenyl)guanidine (m.p. 83—85°C);

N-methoxycarbonyl-N′-(ethoxycarbonylmethyl)-N″-(2-nitro-5-propoxyphenyl)guanidine (m.p.≈120°C, dec.);

N-methoxycarbonyl-N′-(2-(1-piperazinyl)ethyl)-N″-(2-nitro-5-propylthiophenyl)guanidine (m.p. 101—104°C);

N-methoxycarbonyl-N′-(2-hydrogensulfate-ethyl)-N″-(2-nitro-5-propylthiophenyl)guanidine sodium salt (m.p.≈150°C, dec.); and

N-methoxycarbonyl-N'-(2-sulfonic acid ethyl)-N''-(2-nitro-5-propylthiophenyl)guanidine sodium salt (m.p.≈150°C, dec.).

TABLE I

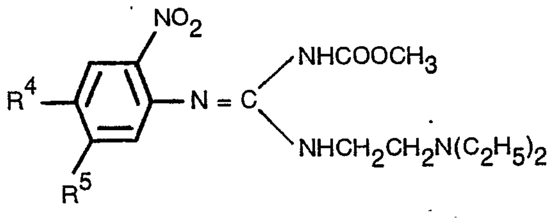

(V)

| Compound No. | $R^4$ | $R^5$ |
|---|---|---|
| V A | $-O \cdot CH_2CH_2CH_3$ | H |
| V B | $-S \cdot CH_2CH_2CH_3$ | H |
| V C | H | $-O \cdot CH_2CH_2CH_3$ |
| V D | H | $-SO_2$ 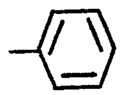 |
| V E | H | $-SO-CH_2CH_2CH_3$ |
| V F | H | $-SO$ 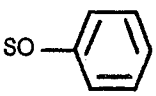 |
| V G | H | $-SCH_2CH_3$ |
| V H | $-O \cdot C(CH_3)_3$ | H |

TABLE II

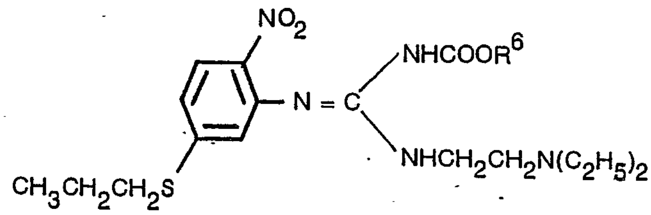

(VI)

| Compound No. | $R^6$ |
| --- | --- |
| VI A | $C_2H_5$ |
| VI B | $n-C_4H_9$ |
| VI C | $-CH_2CH(OCH_3)CH_3$ |
| VI D | $-CH_2CH(OH)CH_3$ |
| VI E | $-CH_2CH_2NH_2$ |

TABLE III

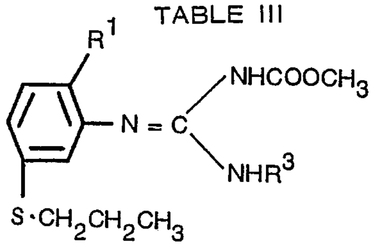

(VII)

| Compound No. | $R^1$ | $R^3$ |
|---|---|---|
| VII A | Cl | $-CH_2CH_2N(C_2H_5)_2$ |
| VII B | 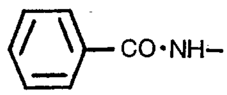 | ,, |
| VII C | $ClCH_2\cdot CO\cdot NH-$ | ,, |
| VII D | 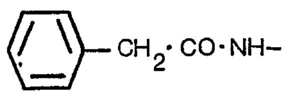 | |
| VII E | 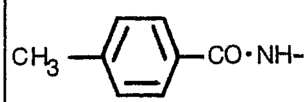 | ,, |
| VII F | 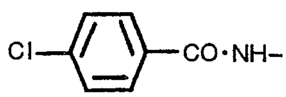 | ,, |
| VII G | $CH_3\cdot SO_2\cdot NH-$ | ,, |
| VII H | $ClCH_2\cdot SO_2\cdot NH-$ | ,, |
| VII I | 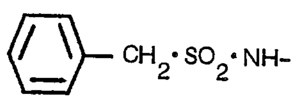 | ,, |
| VII J | 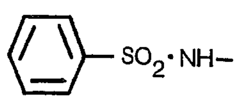 | ,, |
| VII K | 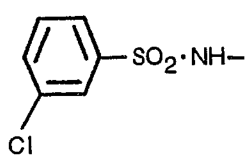 | ,, |
| VII L | 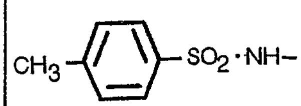 | ,, |

TABLE III (Continued)

| Compound No. | $R^1$ | $R^3$ |
|---|---|---|
| VII M | $NO_2$ | 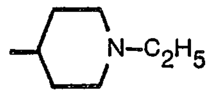 |
| VII N | ,, | 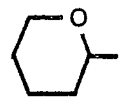 |
| VII O | ,, | 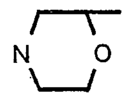 |
| VII P | ,, | 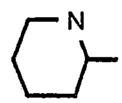 |
| VII Q | $NH_2$ | $-CH_2CH_2OH$ |
| VII R | $-NHCOCH_3$ | $-CH_2CH_2 \cdot S \cdot CH_3$ |
| VII S | $NO_2$ | $-CH_2 \cdot CO \cdot C_2H_5$ |
| VII T | ,, | $-CH_2 \cdot CO \cdot CO \cdot CH_3$ |
| VII U | ,, | $-CH_2-$ (peptide) residue) |
| VII V | $NH_2$ | $-CH_2CH_2NH_2$ |
| VII W | $NO_2$ | 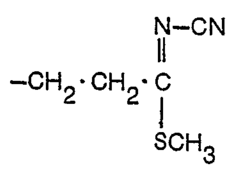 |
| VII X | $NO_2$ | $-CH_2 \cdot COOCH_3$ |

TABLE IV

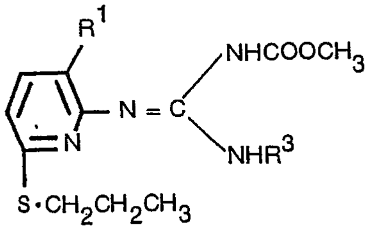

(VIII)

| Compound No. | $R^1$ | $R^3$ |
|---|---|---|
| VIII A | $NO_2$ | $-CH_2 \cdot CH_2N(C_2H_5)_2$ |
| VIII B | ,, | 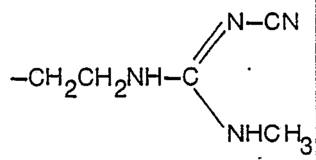 |
| VIII C | ,, | 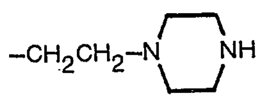 |
| VIII D | $NH_2$ | 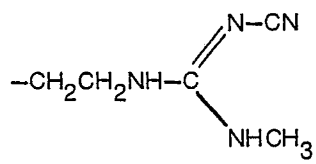 |
| VIII E | $-NH \cdot CO \cdot CH_3$ | 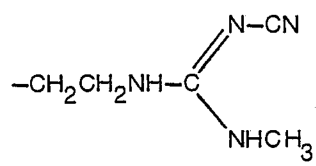 |
| VIII F | $NO_2$ | $-CH_2CH_2COOH$ |
| VIII G | ,, | $-CH_2CH_2CH_2COOH$ |
| VIII H | ,, | $-CH_2COOH$ |

The compounds of the present invention are useful in combatting helminths, i.e. in treating humans and animals suffering from an infestation of parasitic worms, for example, roundworms, hookworms, whipworms or tapeworms, by administering to the host animal a therapeutic amount of a compound of the present invention.

The compounds of this invention exhibit significant anthelmintic effects when administered to a host (e.g. swine, dogs or ruminants) at doses as low as about one milligram per kilogram of body weight per day in dosing over several days, or at about fifty milligrams per kilogram in a single day dosing, according to techniques well known in the art.

The optimum dose for each species of animal and for each type of parasite can readily be determined by one skilled in the art by using standard techniques such as the Modified McMaster Egg Counting Technique as described by H.B. Whitlock and H. McL. Gordon, J. Council Scientific Industrial Research (Australia) 12, p. 50, 1939 H.B. Whitlock J. Council Scientific Research (Australia) 21, p.177, 1948.

From these, and similar tests, anthelmintic efficacy is assessed by determining the number of eggs in faeces passed on the days following treatment with the compound compared with pre-treatment days. In addition, autopsy of animals after treatment will indicate whether the infection has been eradicated. Based on experimentation, proper dosages for curing various infections can be determined.

The compounds of this invention may be administered in suspensions, capsules, feed additives preparations, tablets, as is well known to those skilled in the human and veterinary medical arts. In addition, the compounds may also be used as injectible anthelmintic preparations. For this purpose, the active ingredient is admixed with suitable sterile carriers such as sterile water and isotonic saline solution.

Suitable clinical formulations containing the compounds of this invention can be administered orally in the form of tablets, capsules and elixirs. The active compound is compounded with inert carriers such as, for example, gums, starches and sugars or it may be incorporated into gelatine capsules or formulated into elixirs which have the advantage of being susceptible to manipulations in flavour by the addition of standard, natural or synthetic flavouring agents.

Particularly useful anthelmintic formulations comprising the compounds of this invention for treatment of helminthiasis are either liquid suspensions ready to use or wettable or water-dispersible powders which are mixed with water prior to use.

A liquid-suspension formulation may contain from 50 to 55% w./v. (kg/liter) of the active compound together with a dispersing agent and stabilizing agent. A typical formulation is as follows:

| | |
|---|---|
| N-methoxycarbonyl-N'-(2-diethyl-aminoethyl)-N''-(2-nitro-5-propylthiophenyl) guanidine | 50 to 55 parts by weight |
| Dispersing agent | 1/2 to 2 parts by weight |
| Stabilizing agent | 1 to 3 parts by weight |
| Preservative | as required |
| Water | Sufficient to make 100 volumes. |

Suitable dispersing agents are those containing sulphonate groups, for example sodium lignin sulphonate, or the sulphonated phenol or naphthol formaldehyde polymers. Bentonite may be employed as the stabilizing agent, although it is possible to use such protective colloids as carboxymethyl cellulose and sodium alginate. The formulations can be prepared by mixing the active compound and water containing dissolved dispersing agents very vigourously by means of suitable mechanical mixing equipment.

A wettable or water-dispersible powder formulation may contain about 90 to 95% w./w. of the active compound together with a wetting agent and dispersing agent. A diluent such as kaolin can also be added if a concentration below 98% w./w. is required. An anti-foaming agent and, in some cases, a stabilizing agent may be present. A typical formulation is as follows:

| | |
|---|---|
| N-methoxycarbonyl-N'-(2-diethyl-aminoethyl)-N''-(2-nitro-5-propylthiophenyl)guanidine | 90 to 95 parts by weight |
| Wetting agent | 1/2 to 4 parts by weight |
| Stabilizing agent | 0 to 2 parts by weight |

| | |
|---|---|
| Anti-foaming agent | 0.01 to 1 part by weight |
| Water | 0 to 5 parts by weight |

Suitable wetting agents are the non-ionic alkylphenolethylene oxide adducts, such as an octylphenol or nonylphenol condensed with ten moles of ethylene oxide, or anionic materials, such as the synthetic aryl alkyl sulphonates, or sodium dibutyl naphthalene sulphonate. In general, about 1% w./w. wetting agent is required. The anti-foaming agent employed may be either a silicone or such materials as ethyl hexanol and octanol; and the stabilizing agent may again be chosen from bentonite or the water-soluble gums. Wettable or water-dispersible powder formulations are prepared by careful and adequate mixing of the active compound with other ingredients with or without the addition of some water using typical powder blending equipment such as a ribbon blender. The powder is stirred into water by the user before application in the field.

The following examples show particularly useful formulations:

| A. Tablet formulation | Grams per 1000 tablets |
|---|---|
| N-Methoxycarbonyl-N'-(2-diethylaminoethyl)-N''-(2-nitro-5-propylthiophenyl)guanidine | 200.0 |
| Lactose | 90.0 |
| Dicalcium phosphate, hydrous | 122.5 |
| Polyvinylpyrrolidone | 25.0 |
| Polyethyleneglycol 1500 | 7.5 |
| Corn Starch | 50.0 |
| Magnesium Stearate | 5.0 |
| | 500.0 |

Mix the active compound, the lactose and the dicalcium phosphate. Dissolve the polyethyleneglycol 1500 and the polyvinylpyrrolidone in approximately 20 ml of water. Granulate the powder blend with the water solution, adding additional water if necessary, to produce a damp mass. Pass the wet granulation through a 12 mesh screen; spread on trays and air dry at 35°C. Blend the dry granulates with the starch and the magnesium stearate. Compress the 500 mg tablets.

| B. Capsule formulation | Grams per 1000 capsules |
|---|---|
| N-Methoxycarbonyl-N'-(2-diethylaminoethyl)-N''-(2-nitro-5-propylthiophenyl)guanidine | 200.0 |
| Lactose | 198.0 |
| Magnesium Stearate | 2.0 |
| | 400.0 |

Blend the ingredients and fill into hard gelatine capsules.

| C. Elixir formulation | per 1000 ml |
|---|---|
| N-Methoxycarbonyl-N'-(2-diethylaminoethyl)-N''-(2-nitro-5-propylthiophenyl)guanidine | 40.0 g |
| Sodium citrate | 10.0 g |
| Sugar | 500.0 g |

| | |
|---|---|
| Glycerin | 200.0 g |
| Compound orange spirit | 10.0 ml |
| Alcohol | 100.0 ml |
| Amaranth | 0.1 ml |
| Water to total | 1000.0 ml |

Combine the above ingredients using standard techniques.

| D. Injectible formulation | mg/ml |
|---|---|
| N-Methoxycarbonyl-N'-(2-diethylaminoethyl)-N''-(2-nitro-5-propylthiophenyl)guanidine | 50.0 |
| Polyethylene Glycol 400 | 500.0 |
| Dimethyl Acetamide | 300.0 |
| Benzyl Alcohol | 20.0 |
| Water for Injection to q.s. | 1.0 ml |

Combine the above ingredients using standard techniques.

| E. Injectible formulation | mg/ml |
|---|---|
| N-Methoxycarbonyl-N'-(2-diethylaminoethyl)-N''-(2-nitro-5-propylthiophenyl)guanidine | 100.0 |
| Dimethyl Acetamide | 300.0 |
| Benzyl Alcohol | 20.0 |
| Polyethylene Glycol 400 to q.s. | 1.0 ml |

Combine the above ingredients using standard techniques.

Similarly may be prepared formulations using other compounds of the present invention, e.g. N-methoxycarbonyl-N'-[2-(N'-cyano-N''-methylguanidino)ethyl]-N''-[2-nitro-5-propylthiophenyl]guanidine.

**Claims for the Contracting States: BE CH DE FR GB IT LI LU NL SE**

1. Compounds of the general formula

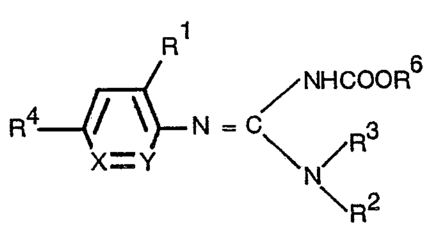

(I) ,

their isomers at the guanidine groups and pharmaceutically acceptable salts of such compounds, wherein

$R^6$ is $C_{1\ to\ 6}$ alkyl or $C_{1\ to\ 6}$ alkyl substituted by $C_{1\ to\ 6}$ alkoxy, hydroxy or amino;

one of X and Y is $CR^5$ and the other is CH or N;

$R^1$ is hydrogen, halogen, nitro, amino, $NHCOR^8$ or $NHSO_2R^8$;

$R^2$ and $R^3$ are independently selected from hydrogen, N-alkyl-piperidyl (wherein the alkyl group has 1 to 4 carbon atoms), tetrahydropyranyl, morpholinyl, piperidyl and $C_1$ to $C_6$ alkyl substituted by one or more substituents selected from OH, $SCH_3$, $COR^9$, $OSO_3H$, $SO_3H$, $COOR^{10}$, piperazinyl, pyridyl and

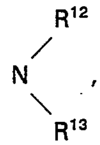

with the proviso that $R^2$ and $R^3$ cannot both be hydrogen;

one of $R^4$ and $R^5$ is selected from hydrogen, $C_1$ to $C_6$ alkyl and $C_1$ to $C_6$ alkoxy and the other from hydrogen, $C_1$ to $C_6$ alkyl and $R^{14}Z$ wherein $R^{14}$ is $C_1$ to $C_6$ alkyl or phenyl and Z represents O, S, $SO_n$ or $SO_2NR^{15}$ with n being 1, 2 or 3 and $R^{15}$ being hydrogen or $C_1$ to $C_6$ alkyl; whereby, in the above definitions,

$R^8$ is $C_1$ to $C_6$ alkyl, $C_1$ to $C_6$ alkyl substituted with phenyl or halogen, phenyl or phenyl substituted with $C_1$ to $C_6$ alkyl or with halogen;

$R^9$ is $C_1$ to $C_6$ alkyl, $C_1$ to $C_6$ acyl or a peptide residue having up to three amino acids;

$R^{10}$ is hydrogen or $C_1$ to $C_6$ alkyl;

$R^{12}$ and $R^{13}$ are independently selected from hydrogen, $C_1$ to $C_6$ alkyl,

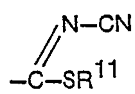 and 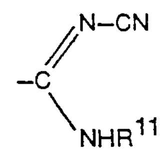

with $R^{11}$ being $C_1$ to $C_6$ alkyl.

2. Compounds according to claim 1 wherein $R^4$ is hydrogen, X is $CR^5$ and Y is CH or N.

3. Compounds according to claim 2 wherein $R^1$ is selected from $NO_2$, $NH_2$ and $NHCOR^8$.

4. Compounds according to claim 3 wherein $R^2$ is hydrogen, $R^3$ represents a substituted $C_1$ to $C_6$ alkyl group and $R^5$ is $R^{14}Z$.

5. Compounds according to claim 4 wherein $R^3$ represents a $C_1$ to $C_6$ alkyl group substituted by $COOR^{10}$, piperazinyl, pyridyl,

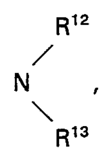

$SO_3H$ or $OSO_3H$.

6. Compounds according to claim 5 having the formula

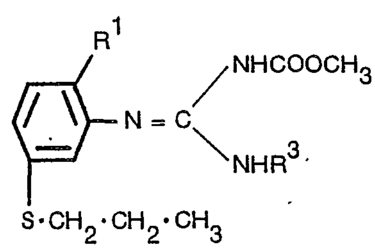

(II) ,

wherein $R^1$ is $NO_2$, $NH_2$ or $NHCOCH_3$ and

$R^3$ is $C_1$ to $C_3$ alkyl substituted by a substituent selected from piperazinyl, COOH, diethylamino,

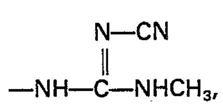

$SO_3H$ and $OSO_3H$.

7. Compounds according to claim 6 being

N-methoxycarbonyl-N'-(2-nitro-5-propylthiophenyl)-N''-[2-(N-piperazinyl)ethyl]guanidine;

N-methoxycarbonyl-N'-[2(N'-cyano-N''-methylguanidino)ethyl]-N-''-[2-nitro-5-propylthiophenyl]guanidine;

N-methoxycarbonyl-N'-(2-nitro-5-propylthiophenyl)-N''-(2-diethylaminoethyl)guanidine; N-methoxycarbonyl-N'-(2-nitro-5-propylthiophenyl)-N''-(2-carboxyethyl)guanidine;

N-methoxycarbonyl-N'-[2-(N'-cyano-N''-methylguanidino)ethyl]-N''-(2-amino-5-propylthiophenyl)guanidine;

N-methoxycarbonyl-N'-[2-(N'-cyano-N''-methylguanidino)ethyl]-N''-[2-acetamido-5-propylthiophenyl]guanidine;

N-methoxycarbonyl-N'-(3-carboxypropyl)-N''-(2-nitro-5-propylthiophenyl)guanidine; and

N-methoxycarbonyl-N'-carboxymethyl-N''-(2-nitro-5-propylthiophenyl)guanidine sodium salt.

8. Pharmaceutical compositions comprising as an active ingredient a compound as defined in any one of claims 1 to 7.

9. Process for the preparation of compounds of formula I as defined in claim 1 characterized in that an isothiourea of formula III

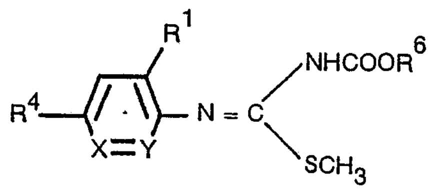

(III)

is reacted with an amine of the formula IV,

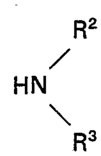

(IV),

whereby in the formulae $R^1$, $R^2$, $R^3$, $R^4$, $R^6$, X and Y are as defined in claim 1, followed, if desired, by transformation into a salt.

10. Process for preparing pharmaceutical compositions as defined in claim 8, characterized in that a compound as defined in any one of claims 1 to 7, is brought into a form suitable for pharmaceutical application.

**Claims for the Contracting State: AT**

1. Process for the preparation of compounds of the general formula

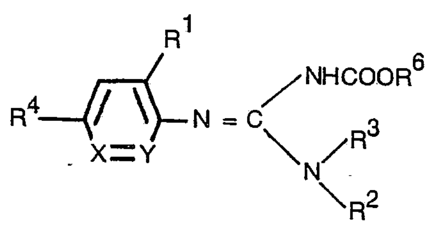

(I),

their isomers at the guanidine groups and pharmaceutically acceptable salts of such compounds, wherein

$R^6$ is $C_1$ to $C_6$ alkyl or $C_1$ to $C_6$ alkyl substituted by $C_1$ to $C_6$ alkoxy, hydroxy or amino;

one of X and Y is $CR^5$ and the other is CH or N;

$R^1$ is hydrogen, halogen, nitro, amino, $NHCOR^8$ or $NHSO_2R^8$;

$R^2$ and $R^3$ are independently selected from hydrogen, N-alkyl-piperidyl (wherein the alkyl group has 1 to 4 carbon atoms), tetrahydropyranyl, morpholinyl, piperidyl and $C_1$ to $C_6$ alkyl substituted by one or more substituents selected from OH, $SCH_3$, $COR^9$, $OSO_3H$, $SO_3H$, $COOR^{10}$, piperazinyl, pyridyl and

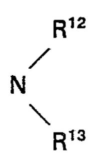

,

with the proviso that $R^2$ and $R^3$ cannot both be hydrogen;

one of $R^4$ and $R^5$ is selected from hydrogen, $C_1$ to $C_6$ alkyl and $C_1$ to $C_6$ alkoxy and the other from hydrogen, $C_1$ to $C_6$ alkyl and $R^{14}Z$ wherein $R^{14}$ is $C_1$ to $C_6$ alkyl or phenyl and Z represents O, S, $SO_n$ or $SO_2NR^{15}$ with n being 1, 2 or 3 and $R^{15}$ being hydrogen or $C_1$ to $C_6$ alkyl; whereby, in the above definitions,

$R^8$ is $C_1$ to $C_6$ alkyl, $C_1$ to $C_6$ alkyl substituted with phenyl or halogen, phenyl or phenyl substituted with $C_1$ to $C_6$alkyl or with halogen;

$R^9$ is $C_1$ to $C_6$ alkyl, $C_1$ to $C_6$ acyl or a peptide residue having up to three amino acids;

$R^{10}$ is hydrogen or $C_1$ to $C_6$ alkyl;

$R^{12}$ and $R^{13}$ are independently selected from hydrogen, $C_1$ to $C_6$ alkyl,

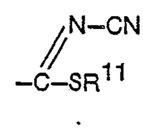 and 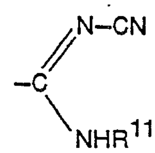

with $R^{11}$ being $C_1$ to $C_6$ alkyl, characterized in that an isothiourea of formula III

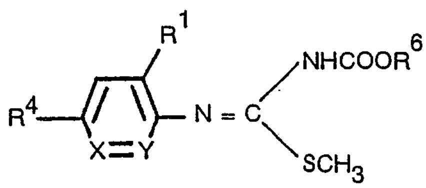 (III)

is reacted with an amine of the formula IV,

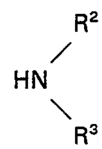 (IV),

whereby in the formulae $R^1$, $R^2$, $R^3$, $R^4$, $R^6$, X and Y are as defined in above, followed, if desired, by transformation into a salt.

2. Process according to claim 1, characterized in that compounds wherein $R^4$ is hydrogen, X is $CR^5$ and Y is CH or N are prepared.

3. Process according to claim 2, characterized in that compounds wherein $R^1$ is $NO_2$, $NH_2$ or $NHCOR^8$ are prepared.

4. Process according to claim 3, characterized in that compounds wherein $R^2$ is hydrogen, $R^3$ represents a substituted $C_1$ to $C_6$ alkyl group and $R^5$ is $R^{14}Z$ are prepared.

5. Process according to claim 4, characterized in that compounds wherein $R^3$ represents a $C_1$ to $C_6$ alkyl group substituted by $COOR^{10}$, piperazinyl, pyridyl, $SO_3H$, $OSO_3H$,

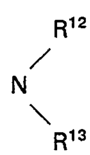

are prepared.

6. Process according to claim 5, characterized in that compounds of the formula

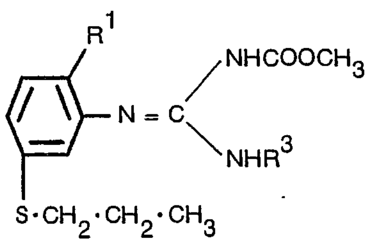

wherein $R^1$ is $NO_2$, $NH_2$ or $NHCOCH_3$ and

$R^3$ is $C_1$ to $C_3$ alkyl substituted by a substituent selected from piperazinyl, $SO_3H$, $OSO_3H$, COOH, diethylamino and

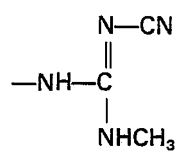

are prepared.

7. Process according to claim 6, characterized in that the compounds,

N-methoxycarbonyl-N'-(2-nitro-5-propylthiophenyl)-N''-[2-(N-piperazinyl)ethyl]guanidine;

N-methoxycarbonyl-N'-[2(N'-cyano-N''-methylguanidino)ethyl]-N''-[2-nitro-5-propylthiophenyl]guanidine;

N-methoxycarbonyl-N'-(2-nitro-5-propylthiophenyl)-N''-(2-diethylaminoethyl)guanidine; N-methoxycarbonyl-N'-(2-nitro-5-propylthiophenyl)-N''-(2-carboxyethyl)guanidine;

N-methoxycarbonyl-N'-[2-(N'-cyano-N''-methylguanidino)ethyl]-N''-(2-amino-5-propylthiophenyl)guanidine;

N-methoxycarbonyl-N'-[2-(N'-cyano-N''-methylguanidino)ethyl]-N''-[2-acetamido-5-propylthiophenyl]guanidine;

N-methoxycarbonyl-N'-(3-carboxypropyl)-N''-(2-nitro-5-propylthiophenyl)guanidine; and

N-methoxycarbonyl-N'-carboxymethyl-N''-(2-nitro-5-propylthiophenyl)guanidine sodium salt are prepared.

8. Process for preparing pharmaceutical compositions, characterized in that a compound as defined in any one of claims 1 to 7 is brought into a form suitable for pharmaceutical administration.

**Patentansprüche für die Vertragsstaaten: BE CH DE FR GB IT LI LU NL SE**

1. Verbindungen der allgemeinen Formel

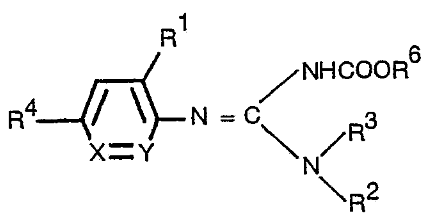

(I),

deren Isomere an den Guanidin-Gruppen sowie pharmazeutisch unbedenkliche Salze solcher Verbindungen, worin

$R^6$ $C_{1-6}$-Alkyl oder durch $C_{1-6}$-Alkoxy, Hydroxy oder Amino substituiertes $C_{1-6}$-Alkyl ist,

einer der Bausteine X und Y $CR^5$ ist und der andere CH oder N ist,

$R^1$ Wasserstoff, Halogen, Nitro, Amino, $NHCOR^8$ oder $NHSO_2R^8$ ist,

$R^2$ und $R^3$ unabhängig voneinander ausgewählt sind aus Wasserstoff, N—Alkylpiperidyl (worin die Alkyl-Gruppe 1 bis 4 Kohlenstoff-Atome hat), Tetrahydropyranyl, Morpholinyl, Piperidyl und $C_1$- bis $C_6$-Alkyl substituiert durch einen oder mehrere Substituenten ausgewählt aus OH, $SCH_3$, $OSO_3H$, $SO_3H$, $COR^9$, $COOR^{10}$, Piperazinyl, Pyridyl und

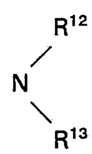

ist, mit der Maßgabe, daß $R^2$ und $R^3$ nicht beide Wasserstoff sein können,

einer der Substituenten $R^4$ und $R^5$ ausgewählt ist aus Wasserstoff, $C_1$- bis $C_6$-Alkyl und $C_1$- bis $C_6$-Alkoxy und der andere ausgewählt ist aus Wasserstoff, $C_1$- bis $C_6$-Alkyl und $R^{14}Z$, worin $R^{14}$ $C_1$- bis $C_6$-Alkyl oder Phenyl ist und Z, O, S, $SO_n$ oder $SO_2NR^{15}$ bezeichnet, wobei n 1, 2 oder 3 ist und $R^{15}$ Wasserstoff oder $C_1$- bis $C_6$-Alkyl ist, wobei in den vorstehenden Definitionen

$R^8$ $C_1$- bis $C_6$-Alkyl, durch Phenyl oder Halogen substituiertes $C_1$- bis $C_6$-Alkyl, Phenyl oder durch $C_1$- bis $C_6$-Alkyl oder Halogen substituiertes Phenyl ist,

$R^9$ $C_1$- bis $C_6$-Alkyl, $C_1$-bis $C_6$-Acyl oder ein Peptid-Rest mit bis zu drei Aminosäuren ist,

$R^{10}$ Wasserstoff oder $C_1$- bis $C_6$-Alkyl ist,

$R^{12}$ und $R^{13}$ unabhängig voneinander ausgewählt sind aus Wasserstoff, $C_1$- bis $C_6$-Alkyl, worin $R^{11}$

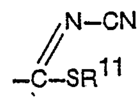 und 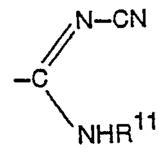 ,

$C_1$- bis $C_6$-Alkyl ist.

2. Verbindungen nach Anspruch 1, dadurch gekennzeichnet, daß $R^4$ Wasserstoff ist, X $CR^5$ ist und Y CH oder N ist.

3. Verbindungen nach Anspruch 2, dadurch gekennzeichnet, daß $R^1$ ausgewählt ist aus $NO_2$, $NH_2$ und $NHCOR^8$.

4. Verbindungen nach Anspruch 3, dadurch gekennzeichnet, daß $R^2$ Wasserstoff ist, $R^3$ eine substituierte $C_1$- bis $C_6$-Alkyl-Gruppe bezeichnet und $R^5$ $R^{14}Z$ ist.

5. Verbindungen nach Anspruch 4, dadurch gekennzeichnet, daß $R^3$ eine $C_1$- bis $C_6$-Alkyl-Gruppe bezeichnet, die durch $COOR^{10}$, Piperazinyl, Pyridyl,

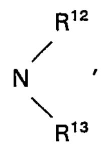 ,

$SO_3H$ oder $OSO_3H$ substituiert ist.

6. Verbindungen nach Anspruch 5 mit der Formel

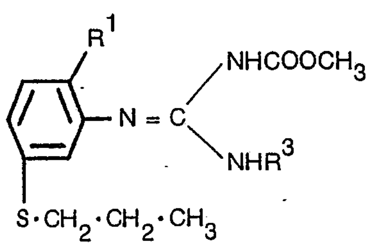 (II) ,

in der

$R^1$ $NO_2$, $NH_2$ oder $NHCOCH_3$ ist und

$R^3$ $C_1$- bis $C_3$-Alkyl ist, das durch einen Substituenten ausgewählt aus Piperazinyl, COOH, Diethylamino,

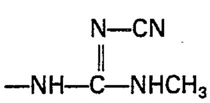 ,

$SO_3H$ und $OSO_3H$ substituiert ist.

7. Verbindungen nach Anspruch 6, nämlich

N-Methoxycarbonyl-N'-(2-nitro-5-propylthiophenyl)-N''-[2-(N-piperazinyl)ethyl]guanidin,

N-Methoxycarbonyl-N'-[2-(N'-cyano-N''-methylguanidino)ethyl]-N''-(2-nitro-5-propylthiophenyl)guanidin,

N-Methoxycarbonyl-N'-(2-nitro-5-propylthiophenyl)-N''-(2-diethyl-aminoethyl)guanidin,

N-Methoxycarbonyl-N'-(2-nitro-5-propylthiophenyl)-N''-(2-carboxyethyl)guanidin,

N-Methoxycarbonyl-N'-[2-(N'-cyano-N''-methylguanidino)ethyl]-N''-(2-amino-5-propylthiophenyl)guanidin,

N-Methoxycarbonyl-N'-[2-(N'-cyano-N''-methylguanidino)ethyl]-N''-(2-acetamido-5-propylthiophenyl)guanidin,

N-Methoxycarbonyl-N'-(3-carboxypropyl)-N''-(2-nitro-5-propylthiophenyl)guanidin und

N-Methoxycarbonyl-N'-carboxymethyl-N''-(2-nitro-5-propylthiophenyl)guanidin-Natrium-Salz.

8. Pharmazeutische Zusammensetzungen, enthaltend als Wirkstoff eine Verbindung nach irgendeinem der Ansprüche 1 bis 7.

9. Verfahren zur Herstellung von Verbindungen der Formel I nach Anspruch 1, dadurch gekennzeichnet, daß ein Isothioharnstoff der Formel III

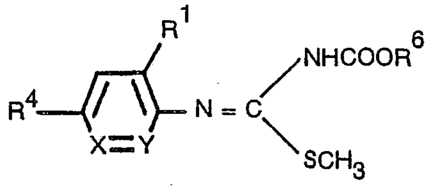

(III)

mit einem Amin der Formel IV

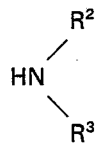

(IV)

umgesetzt wird, wobei in den Formeln $R^1$, $R^2$, $R^3$, $R^4$, $R^6$, X und Y die in Anspruch 1 angegebenen Bedeutungen haben, gewünschtenfalls gefolgt von der Überführung in ein Salz.

10. Verfahren zur Herstellung von pharmazeutischen Zusammensetzungen nach Anspruch 8, dadurch gekennzeichnet, daß eine Verbindung nach irgendeinem der Ansprüche 1 bis 7 in eine für die pharmazeutische Anwendung geeignete Form gebracht wird.

**Patentansprüche für den Vertragsstaat: AT**

1. Verfahren zur Herstellung von Verbindungen der allgemeinen Formel

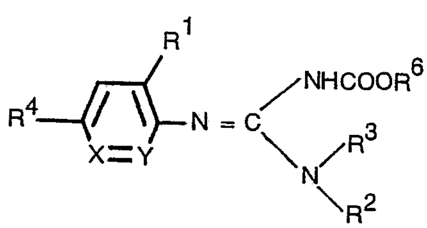

(I),

deren Isomerer an den Guanidin-Gruppen sowie pharmazeutisch unbedenklicher Salze solcher Verbindungen, worin

$R^6$ $C_{1-6}$-Alkyl oder durch $C_{1-6}$-Alkoxy, Hydroxy oder Amino substituiertes $C_{1-6}$-Alkyl ist,

einer der Bausteine X und Y $CR^5$ ist und der andere CH oder N ist,

$R^1$ Wasserstoff, Halogen, Nitro, Amino, $NHCOR^8$ oder $NHSO_2R^8$ ist,

$R^2$ und $R^3$ unabhängig voneinander ausgewählt sind aus Wasserstoff, N—Alkylpiperidyl (worin die Alkyl-Gruppe 1 bis 4 Kohlenstoff-Atome hat), Tetrahydropyranyl, Morpholinyl, Piperidyl und $C_1$- bis $C_6$-Alkyl substituiert durch einen oder mehrere Substituenten ausgewählt aus OH, $SCH_3$, $OSO_3H$, $SO_3H$, $COR^9$, $COOR^{10}$, Piperazinyl, Pyridyl und

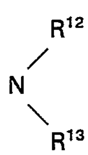

ist, mit der Maßgabe, daß $R^2$ und $R^3$ nicht beide Wasserstoff sein können,

einer der Substituenten $R^4$ und $R^5$ ausgewählt ist aus Wasserstoff, $C_1$- bis $C_6$-Alkyl und $C_1$- bis $C_6$-Alkoxy und der andere ausgewählt ist aus Wasserstoff, $C_1$- bis $C_6$-Alkyl und $R^{14}Z$, worin $R^{14}$ $C_1$- bis $C_6$-Alkyl oder Phenyl ist und Z, O, S, $SO_n$ oder $SO_2NR^{15}$ bezeichnet, wobei n 1, 2 oder 3 ist und $R^{15}$ Wasserstoff oder $C_1$- bis $C_6$-Alkyl ist, wobei in den vorstehenden Definitionen

$R^8$ $C_1$- bis $C_6$-Alkyl, durch Phenyl oder Halogen substituiertes $C_1$- bis $C_6$-Alkyl, Phenyl oder durch $C_1$- bis $C_6$-Alkyl oder Halogen substituiertes Phenyl ist,

$R^9$ $C_1$- bis $C_6$-Alkyl, $C_1$-bis $C_6$-Acyl oder ein Peptid-Rest mit bis zu drei Aminosäuren ist,

$R^{10}$ Wasserstoff oder $C_1$- bis $C_6$-Alkyl ist,
$R^{12}$ und $R^{13}$ unabhängig voneinander ausgewählt sind aus Wasserstoff, $C_1$- bis $C_6$-Alkyl, worin $R^{11}$

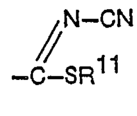 und 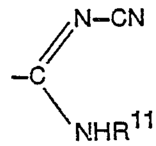,

$C_1$- bis $C_6$-Alkyl ist, dadurch gekennziechnet, daß ein Isothioharnstoff der Formel (III)

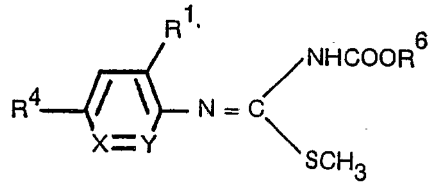

(III)

mit einem Amin der Formel (IV)

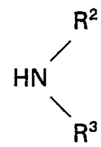

(IV)

umgesetzt wird, wobei in den Formeln $R^1$, $R^2$, $R^3$, $R^4$, $R^6$, X und Y die im Vorstehenden angegebenen Bedeutungen haben, gewünschtenfalls gefolgt von der Überführung in ein Salz.

2. Verfahren nach Anspruch 1, dadurch gekennzeichnet, daß Verbindungen, in denen $R^4$ Wasserstoff ist, X $CR^5$ ist und Y CH oder N ist, hergestellt werden.

3. Verfahren nach Anspruch 2, dadurch gekennzeichnet, daß Verbindungen, in denen $R^1$ $NO_2$, $NH_2$ oder $NHCOR^8$ ist, hergestellt werden.

4. Verfahren nach Anspruch 3, dadurch gekennzeichnet, daß Verbindungen, in denen $R^2$ Wasserstoff ist, $R^3$ eine substituierte $C_1$- bis $C_6$-Alkyl-Gruppe bezeichnet und $R^5$ $R^{14}Z$ ist, hergestellt werden.

5. Verfahren nach Anspruch 4, dadurch gekennzeichnet, daß Verbindungen, in denen $R^3$ eine $C_1$- bis $C_6$-Alkyl-Gruppe bezeichnet, die durch $COOR^{10}$, Piperazinyl, Pyridyl,

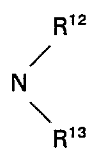

$SO_3H$ oder $OSO_3H$ substituiert ist, hergestellt werden.

6. Verfahren nach Anspruch 5, dadurch gekennzeichnet, daß Verbindungen mit der Formel

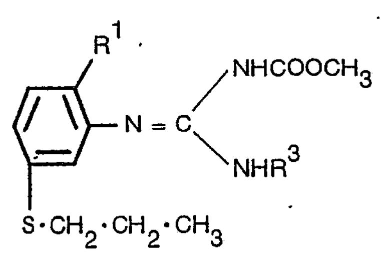,

in der
$R^1$ $NO_2$, $NH_2$ oder $NHCOCH_3$ ist und
$R^3$ $C_1$- bis $C_3$-Alkyl ist, das durch einen Substituenten ausgewählt aus Piperazinyl, $SO_3H$, $OSO_3H$, COOH, Diethylamino und

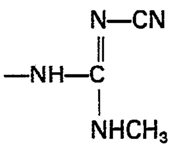

substituiert ist, hergestellt werden.

7. Verfahren nach Anspruch 6, dadurch gekennzeichnet, daß die Verbindungen
N-Methoxycarbonyl-N'-(2-nitro-5-propylthiophenyl)-N''-[2-(N-piperazinyl)ethyl]guanidin,
N-Methoxycarbonyl-N'-[2-(N'-cyano-N''-methylguanidino)ethyl]-N''-(2-nitro-5-propylthiophenyl)guanidin,
N-Methoxycarbonyl-N'-(2-nitro-5-propylthiophenyl)-N''-(2-diethyl-aminoethyl)guanidin,
N-Methoxycarbonyl-N'-(2-nitro-5-propylthiophenyl)-N''-(2-carboxyethyl)guanidin,
N-Methoxycarbonyl-N'-[2-(N'-cyano-N''-methylguanidino)ethyl]-N''-(2-amino-5-propylthiophenyl)guanidin,
N-Methoxycarbonyl-N'-[2-(N'-cyano-N''-methylguanidino)ethyl]-N''-(2-acetamido-5-propylthiophenyl)guanidin,
N-Methoxycarbonyl-N'-(3-carboxypropyl)-N''-(2-nitro-5-propylthiophenyl)guanidin und
N-Methoxycarbonyl-N'-carboxymethyl-N''-(2-nitro-5-propylthiophenyl)guanidin-Natrium-Salz
hergestellt werden.

8. Verfahren zur Herstellung pharmazeutischer Zusammensetzungen, dadurch gekennzeichnet, daß eine Verbindung nach irgendeinem der Ansprüche 1 bis 7 in eine für die pharmazeutische Verabreichung geeignete Form gebracht wird.

**Revendications pour les Etats contractants: BE CH DE FR GB IT LI LU NL SE**

1. Composés caractérisés en ce qu'ils sont représentés par la formule générale

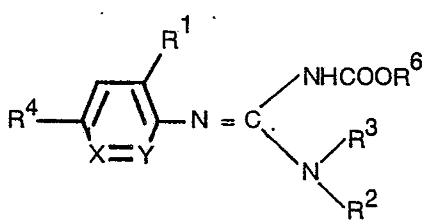

(I),

leurs isomères aux groupes guanidines et des sels pharmaceutiquement acceptables de tels composés, dans laquelle

$R^6$ est alkyle $C_1$ à $C_6$ ou alkyle $C_1$ à $C_6$ substitué par alkoxy $C_1$ à $C_6$, hydroxy ou amino;

un des X et Y est $CR^5$ et l'autre est CH ou N;

$R^1$ est hydrogène, halogène, nitro, amino, $NHCOR^8$ ou $NHSO_2R^8$;

$R^2$ et $R^3$ sont indépendamment choisis parmi hydrogène, N-alkyl-pipéridyle (dans lequel le groupe alkyle a 1 à 4 atomes de carbone), tétrahydropyranyle, morpholinyle, pipéridyle et alkyle $C_1$ à $C_6$ substitué par un ou plusieurs substituants choisis parmi OH, $SCH_3$, $COR^9$, $OSO_3H$, $SO_3H$, $COOR^{10}$, pipérazinyle, pyridyle et

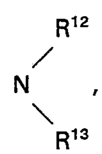

,

à condition que $R^2$ et $R^3$ ne soient pas simultanément l'hydrogène;

un des $R^4$ et $R^5$ est choisi parmi hydrogène, alkyle $C_1$ à $C_6$ et alkoxy $C_1$ à $C_6$ et l'autre parmi hydrogène, alkyle $C_1$ à $C_6$ et $R^{14}Z$ dans lequel $R^{14}$ est alkyle $C_1$ à $C_6$ ou phényle et Z représente O, S, $SO_n$ ou $SO_2NR^{15}$, n valant 1, 2 ou 3 et $R^{15}$ étant hydrogène ou alkyle $C_1$ à $C_6$; dans les définitions ci-dessus;

$R^8$ est alkyle $C_1$ à $C_6$, alkyle $C_1$ à $C_6$ substitué par phényle ou halogène, phényle ou phényle substitué par alkyle $C_1$ à $C_6$ ou par halogène;

$R^9$ est alkyle $C_1$ à $C_6$, acyle $C_1$ à $C_6$ ou un résidu peptide ayant jusqu'à trois amino acides;

$R^{10}$ est hydrogène ou alkyle $C_1$ à $C_6$;

$R^{12}$ et $R^{13}$ sont indépendamment choisi parmi hydrogène, alkyle $C_1$ à $C_6$,

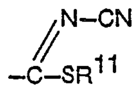 et 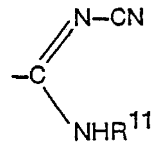

$R^{11}$ étant alkyle $C_1$ à $C_6$.

2. Composés suivant la revendication 1, caractérisés en ce que $R^4$ est l'hydrogène, X est $CR^5$ et Y est CH ou N.

3. Composés suivant la revendication 2, caractérisés en ce que $R^1$ est choisi parmi $NO_2$, $NH_2$ et $NHCOR^8$.

4. Composés suivant la revendication 3, caractérisés en ce que $R^2$ est l'hydrogène, $R^3$ représente un groupe alkyle $C_1$ à $C_6$ substitué et $R^5$ est $R^{14}Z$.

5. Composés suivant la revendication 4, caractérisés en ce que $R^3$ représente un groupe alkyle $C_1$ à $C_6$ substitué par $COOR^{10}$, pipérazinyle, pyridyle,

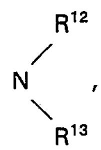 ,

$SO_3H$ ou $OSO_3H$.

6. Composés suivant la revendication 5, caractérisés en ce qu'ils sont représentés par la formule

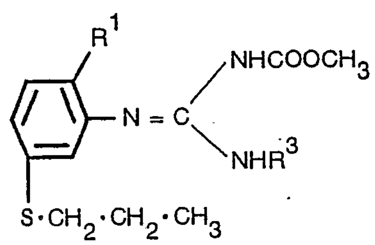 (II) ,

dans laquelle

$R^1$ est $NO_2$, $NH_2$ ou $NHCOCH_3$ et

$R^3$ est alkyle $C_1$ à $C_3$ substitué par un substituant choisi parmi pipérazinyle, COOH, diéthylamino,

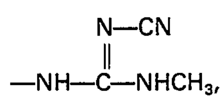

$SO_3H$ et $OSO_3H$.

7. Composés suivant la revendication 6, caractérisés en ce qu'ils sont:

N-méthoxycarbonyl-N'-(2-nitro-5-propylthiophényl)-N''-[2-(N-pipérazinyl)éthyl]guanidine;

N-méthoxycarbonyl-N'-[2(N'-cyano-N''-méthylguanidino)éthyl]-N''-[2-nitro-5-propylthiophényl]guanidine;

N-méthoxycarbonyl-N'-(2-nitro-5-propylthiophényl)-N''-(2-diéthylaminoéthyl)guanidine;

N-méthoxycarbonyl-N'-(2-nitro-5-propylthiophényl)-N''-(2-carboxyéthyl)guanidine;

N-méthoxycarbonyl-N'-[2-(N'-cyano-N''-méthylguanidino)éthyl]-N''-(2-amino-5-propylthiophényl)guanidine;

N-méthoxycarbonyl-N'-[2-(N'-cyano-N''-méthylguanidino)éthyl]-N''-[2-acétamido-5-propylthiophényl]guanidine;

N-méthoxycarbonyl-N'-(3-carboxypropyl)-N''-(2-nitro-5-propylthiophényl)guanidine; et

sel sodique de N-méthoxycarbonyl-N'-carboxyméthyl-N''-(2-nitro-5-propylthiophényl)guanidine.

8. Compositions pharmaceutiques caractérisées en ce qu'elles comprennent comme ingrédient actif un composé comme défini dans n'importe laquelle des revendications 1 à 7.

9. Procédé de préparation de composés de formule I, comme définis dans la revendication 1, caractérisé en ce qu'on fait réagir une isothiourée de formule III

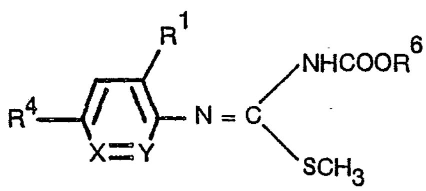

(III)

avec une amine de formule IV,

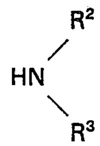

(IV)

dans lesquelles $R^1$, $R^2$, $R^3$, $R^4$, $R^6$, X et Y sont comme définis dans la revendication 1, puis, si on le désire, transforme en un sel.

10. Procédé de préparation de compositions pharmaceutiques commes définies dans la revendication 8, caractérisé en ce qu'un composé suivant n'importe laquelle des revendications 1 à 7 est mis sous une forme appropriée pour application pharmaceutique.

**Revendications pour l'Etat contractant: AT**

1. Procédé de préparation de composés de formule générale

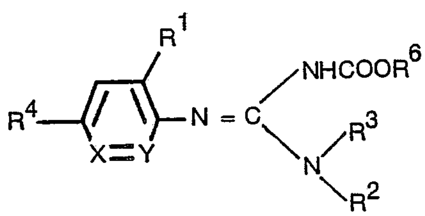

(I) ,

de leurs isomères aux groupes guanidines et des sels pharmaceutiquement acceptables de ces composés, dans laquelle

$R^6$ est alkyle $C_1$ à $C_6$ ou alkyle $C_1$ à $C_6$ substitué par alkoxy $C_1$ à $C_6$, hydroxy ou amino;

un des X et Y est $CR^5$ et l'autre est CH ou N;

$R^1$ est hydrogène, halogène, nitro, amino, $NHCOR^8$ ou $NHSO_2R^8$;

$R^2$ et $R^3$ sont indépendamment choisis parmi hydrogène, N-alkyl-pipéridyle (dans lequel le groupe alkyle a 1 à 4 atomes de carbone), tétrahydropyranyle, morpholinyle, pipéridyle et alkyle $C_1$ à $C_6$ substitué par un ou plusieurs substituants choisis parmi OH, $SCH_3$, $COR^9$, $OSO_3H$, $SO_3H$, $COOR^{10}$, pipérazinyle, pyridyle et

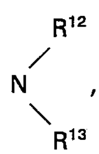

,

à condition que $R^2$ et $R^3$ ne soient pas simultanément l'hydrogène;

un des $R^4$ et $R^5$ est choisi parmi hydrogène, alkyle $C_1$ à $C_6$ et alkoxy $C_1$ à $C_6$ et l'autre parmi hydrogène, alkyle $C_1$ à $C_6$ et $R^{14}Z$ dans lequel $R^{14}$ est alkyle $C_1$ à $C_6$ ou phényle et Z représente O, S, $SO_n$ ou $SO_2NR^{15}$, n valant 1, 2 ou 3 et $R^{15}$ étant hydrogène ou alkyle $C_1$ à $C_6$; dans les définitions ci-dessus;

$R^8$ est alkyle $C_1$ à $C_6$, alkyle $C_1$ à $C_6$ substitué par phényle ou halogène, phényle ou phényle substitué par alkyle $C_1$ à $C_6$ ou par halogène;

$R^9$ est alkyle $C_1$ à $C_6$, acyle $C_1$ à $C_6$ ou un résidu peptide ayant jusqu'à trois amino acides;

$R^{10}$ est hydrogène ou alkyle $C_1$ à $C_6$;

$R^{12}$ et $R^{13}$ sont indépendamment choisi parmi hydrogène, alkyle $C_1$ à $C_6$,

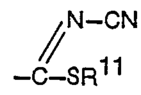

et

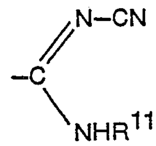

,

$R^{11}$ étant alkyle $C_1$ à $C_6$, caractérisé em ce qu'on fait réagir une isothiourée de formule III

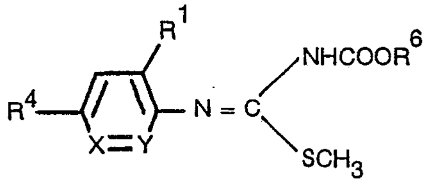

(III)

avec une amine de formule IV,

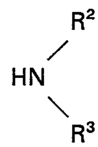

(IV)

formules dans lesquelles $R^1$, $R^2$, $R^3$, $R^4$, $R^6$, X et Y sont comme définis ci-dessus, puis, si on le désire, transforme en un sel.

2. Procédé suivant la revendication 1, caractérisé en ce qu'on prépare des composés dans lesquels $R^4$ est hydrogène, X est $CR^5$ et Y est CH ou N.

3. Procédé suivant la revendication 2, caractérisé en ce qu'on prépare des composés dans lesquels $R^1$ est $NO_2$, $NH_2$ ou $NHCOR^8$.

4. Procédé suivant la revendication 3, caractérisé en ce qu'on prépare des composés dans lesquels $R^2$ est hydrogène, $R^3$ représente un groupe alkyle $C_1$ à $C_6$ substitué et $R^5$ est $R^{14}Z$.

5. Procédé suivant la revendication 4, caractérisé en ce qu'on prépare des composés dans lesquels $R^3$ représente un groupe alkyle $C_1$ à $C_6$ substitué par $COOR^{10}$, pipérazinyle, pyridyle, $SO_3H$, $OSO_3H$,

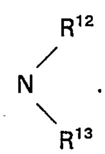

.

6. Procédé suivant la revendication 5, caractérisé en ce qu'on prépare des composés de formule

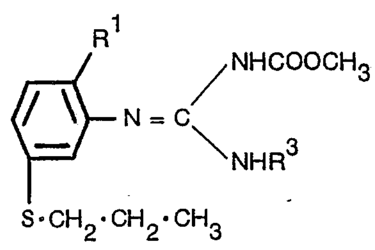

dans laquelle

$R^1$ est $NO_2$, $NH_2$ ou $NHCOCH_3$ et

$R^3$ est alkyle $C_1$ à $C_3$ substitué par un substituant choisi parmi pipérazinyle, $SO_3H$, $OSO_3H$, COOH, diéthylamino et

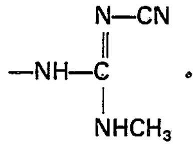

7. Procédé suivant la revendication 6, caractérisé en ce qu'on prépare les composés suivants:

N-méthoxycarbonyl-N'-(2-nitro-5-propylthiophényl)-N''-[2-(N-pipérazinyl)éthyl]guanidine;

N-méthoxycarbonyl-N'-[2(N'-cyano-N''-méthylguanidino)éthyl]-N''-[2-nitro-5-propylthiophényl]guanidine;

N-méthoxycarbonyl-N'-(2-nitro-5-propylthiophényl)-N''-(2-diéthylaminoéthyl)guanidine;

N-méthoxycarbonyl-N'-(2-nitro-5-propylthiophényl)-N''-(2-carboxyéthyl)guanidine;

N-méthoxycarbonyl-N'-[2-(N'-cyano-N''-méthylguanidino)éthyl]-N''-(2-amino-5-propylthiophényl)guanidine;

N-méthoxycarbonyl-N'-[2-(N'-cyano-N''-méthylguanidino)éthyl]-N''-[2-acétamido-5-propylthiophényl]guanidine;

N-méthoxycarbonyl-N'-(3-carboxypropyl)-N''-(2-nitro-5-propylthiophényl)guanidine; et

sel sodique de N-méthoxycarbonyl-N'-carboxyméthyl-N''-(2-nitro-5-propylthiophényl)guanidine.

8. Procédé de préparation de compositions pharmaceutiques caractérisé en ce qu'on composé suivant n'importe laquelle des revendications 1 à 7 est mis sous une forme appropriée pour administration pharmaceutique.